# EUROPÄISCHE PATENTSCHRIFT

(11) **EP 1 423 436 B1**
(45) Veröffentlichungstag und Bekanntmachung des Hinweises auf die Patenterteilung: **12.04.2006**
(21) Anmeldenummer: 02796214.1
(22) Anmeldetag: 09.08.2002
(51) Int. Cl.: C08B 37/10, A61P 19/02, A61P 7/02

(54) **VERWENDUNG VON HEPARINOID-DERIVATEN ZUR BEHANDLUNG UND DIAGNOSE VON MIT HEPARINOIDEN BEHANDELBAREN ERKRANKUNGEN**
USE OF HEPARINOID DERIVATIVES FOR TREATING AND DIAGNOSING DISEASES THAT CAN BE TREATED BY HEPARINOIDS
UTILISATION DE DERIVES D'HEPARINOIDES POUR TRAITER ET DIAGNOSTIQUER DES MALADIES POUVANT ETRE TRAITEES A L'AIDE D'HEPARINOIDES

(30) Priorität: 22.08.2001 DE 10141106
(43) Veröffentlichungstag der Anmeldung: 02.06.2004
(73) Patentinhaber: Sanofi-Aventis Deutschland GmbH, 65929 Frankfurt am Main (DE)
(72) Erfinder: ULMER, Wolfgang, 65817 Eppstein (DE); JURETSCHKE, Hans-Paul, 63303 Dreieich (DE); KERN, Christopher, 67480 Edenkoben (DE)
(86) Internationale Anmeldenummer: PCT/EP2002/008909
(87) Internationale Veröffentlichungsnummer: WO 2003/018640

(56) Entgegenhaltungen:
- WO-A-99/55230
- US-A- 5 145 841
- US-A- 5 155 215

## Beschreibung

Die Erfindung betrifft Heparinoid-Derivate, Verfahren zu deren Herstellung und deren Verwendung sowohl bei der Therapie als auch zu diagnostischen Zwecken, zur Lokalisation der eingesetzten Dosis, wie auch zur Überwachung des Behandlungserfolges von Erkrankungen wie Thrombose und Osteoarthrose.

Heparin ist ein aus tierischen Organen isolierbares, in Mastzellen synthetisiertes hochsulfoniertes Glykosaminoglykan bestehend aus D-Glucosamin und D-Glucuronsäure, mit einem Molekulargewicht von etwa 17 000 Dalton.

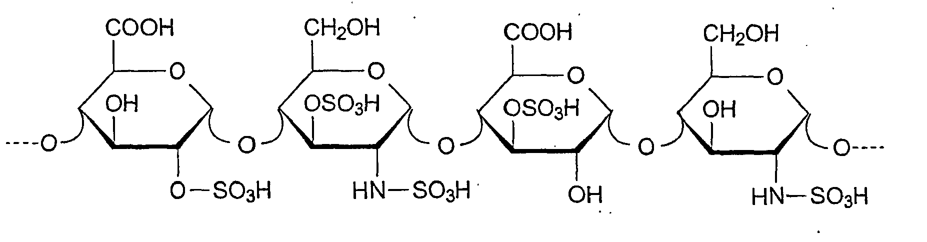

D-Glucosamin und D-Glucuronsäure sind dabei jeweils α-1,4-glykosidisch zum Disaccharid verbunden und bilden die Heparin-Untereinheiten, die miteinander ebenfalls α-1,4-glykosidisch zum Heparin verknüpft sind. Die Stellung der SulfoGruppen kann wechseln; eine Tetrasaccharid-Einheit enthält 4 bis 5 Schwefelsäure-Reste. Heparansulfat (Heparitinsulfat) enthält weniger O- und N-gebundene Sulfo-, dafür jedoch N-Acetyl-Gruppen. Man kann Heparin als anionischen Polyelektrolyt auffassen. Heparin kommt, gebunden an Proteine, besonders in der Leber (griechisch: hepar) vor und verhindert als Antikoagulans das Gerinnen des im Körper kreisenden Blutes. Heparansulfat findet sich als Bestandteil von Proteoglykanen (Perlecan) auf den Zeiloberflächen und in der extrazellulären Matrix vieler Gewebe. Heparin verstärkt die inhibitorische Wirkung des Antithrombins III auf Thrombin, wodurch die Katalyse der Umwandlung von Fibrinogen zu Fibrin durch Thrombin unterbunden wird, sowie auf verschiedene andere Blutgerinnungs-Faktoren; beispielsweise wird auch die Umwandlung von Prothrombin in Thrombin verhindert und der Abbau von Lipoproteinen durch Lipoprotein-Lipase aktiviert.

Heparinoid ist eine Sammelbezeichnung für alle Stoffe, die wie Heparin wirken. Hierher gehören auch Pentosan-Polysulfat, Xylan-, Dextran- oder Chitinsulfate, Di-, Tri-, oder Oligomere und Polymere aus Iduron/Uronsäuren und/oder Glucosamin, Oligo- oder Polysaccharide aus Pentose- und/oder Hexose-Bausteinen und/oder Mannitol in zufälliger oder regelmäßiger Anordnung, Heparan-, Heparitin-, Keratan- oder Dermatansulfate, Hyaluronsäure, Chondroitinsulfat-A, B oder C, unfraktioniertes Heparin wie auch fraktioniertes Heparin, Enoxaparin, Nadroparin (Fraxiparin), Dalteparin (Fragmin), Bemiparin, Tinzaparin, Ardeparin, Low Molecular Weight Heparin (LMWH), Ultra Low Molecular Weight Heparin (ULMWH) oder damit vergleichbare synthetische Polysaccharide, sowie deren Salze sowie verknüpfte und quervernetzte Ketten (Di-, Tri- oder Oligomere) der oben genannten Verbindungen, sowie Heparinoide mit daran gebundenen Peptiden, Proteinen, Lipiden oder Nukleinsäuren.

Enoxaparin ist ein zur Klasse der niedermolekularen Heparine (LMWH) gehörender, international durch Patente geschützter Wirkstoff von Aventis Pharma. (US 5,389,618) Die Anwendung von Enoxaparin zur anti-thrombotischen Therapie ist etablierter Stand der Technik.
Enoxaparin-Na ist das Natrium-Salz von niedermolekularem Heparin, das durch alkalische Depolymerisation des Benzylesterderivates von Heparin aus Schweine-Intestinal-Mucosa gewonnen wird. Die Hauptmenge der Komponenten trägt eine 4-Enopyranose-uronat-Struktur am nicht-reduzierenden Ende ihrer Kette.
Die durchschnittliche Molekülmasse beträgt etwa 4500 Dalton.
Der prozentuale Anteil von Molekülen kleiner 2000 Dalton liegt zwischen 12% und 20%. Der Massenprozent-Anteil der Ketten einer Größe zwischen 2000 und 8000 Dalton liegt zwischen 68% und 88% in bezug auf den European Pharmacopoeia calibration reference Standard für niedermolekulare Heparine. Der Sulfatierungsgrad liegt durchschnittlich bei 2 Resten pro Disaccharid-Einheit.
Die Enoxaparin-Polysaccharid-Kette ist wie bei Heparin aus alternierenden Einheiten von sulfatierten Glucosaminen und Uron-Säuren, die durch glycosidische Bindungen verknüpft sind, zusammengesetzt. Die Struktur unterscheidet sich von Heparin beispielsweise dadurch, dass durch den Depolymerisierungsprozess eine Doppelbindung am nicht-reduzierenden Ende der Kette entsteht. Enoxaparin kann von Heparin unterschieden werden durch UV-Spektroskopie und durch das ¹³C nuclear magnetic resonance Spektrum, welche die Doppelbindung im terminalen Ring zeigen, sowie durch high performance size exclusion chromatography.

Im Krankheitsbild der Osteoarthrose stellt die Degradation des Aggrecans, des Hauptproteoglykans des artikulären Knorpels, ein sehr frühes und entscheidendes Ereignis dar. Der pathologische Verlust des Knorpel-Aggrecans resultiert aus proteolytischen Spaltungen in dessen interglobulärer Domäne. Aminosäuresequenzanalysen von Proteoglykanmetaboliten, isoliert aus der Synovialflüssigkeit von Patienten, die an einer Gelenkschädigung, an Osteoarthrose oder an einer entzündlichen Gelenkerkrankung leiden, zeigten, dass eine proteolytische Spaltung bevorzugt zwischen den Aminosäuren Glu³⁷³ und Ala³⁷⁴ in der interglobulären Domäne des humanen Aggrecans stattfindet (Lohmander et al., Arthritis Rheum. 36, (1993), 1214-1222). Die proteolytische Aktivität, die für diese Spaltung verantwortlich ist, wird als "Aggrecanase" bezeichnet und kann zur Superfamilie der Metalloproteinasen (MP) gerechnet werden.

Bei den Metalloproteinasen ist Zink im katalytisch aktiven Zentrum essentiell. MP spalten Kollagen, Laminin, Proteoglykane, Elastin oder Gelatin unter physiologischen Bedingungen und spielen daher eine wichtige Rolle im Knochen und Bindegewebe.
Eine Vielzahl von verschiedenen MP-Inhibitoren sind bekannt (J.S.Skotnicki et al., Ann. N.Y. Acad. Sci. 878, 61-72 [1999]; EP 0 606 046; WO94/28889).

Einige dieser Inhibitoren sind bezüglich ihrer Spezifität wenig charakterisiert; andere sind mehr oder weniger selektiv vor allem gegen Matrix-Metalloproteinasen (MMPs) gerichtet.

Aggrecanase unterscheidet sich von den Matrix-Metalloproteinasen (MMPs) durch ihre andersartige, gegen bestimmte, im Aggrecan vorkommende und von MMPs nicht bevorzugte Spaltstellen gerichtete Spezifität. Durch die Spaltung entstehen charakteristische Fragmente, die unter Verwendung geeigneter Antikörper nachgewiesen werden können.

In früheren Arbeiten wurde gefunden, dass Enoxaparin dosisabhängig die Aggrecanase-Aktivität in Synovialflüssigkeit hemmt und deshalb, intra-artikulär appliziert, zur Therapie von Osteoarthrose eingesetzt werden kann (DE 100 63 006.5).

Ein inhärenter Nachteil von Heparinoiden (Polysacchariden) besteht jedoch darin, dass diese in ihrer chemischen Struktur keinen analytisch nutzbaren Chromophor enthalten. Dies hat zur Folge, dass die analytische Überwachung therapeutisch wirksamer Konzentrationen durch gängige Analysemethoden wie HochdruckflüssigkeitsChromatographie (HPLC) auf erhebliche Probleme stößt.
Zur Überwachung der antithrombotischen Therapie mit Heparinoiden kann in der Regel mit ausreichender Empfindlichkeit nur deren biologische Wirkung, die durch Heparinoide katalysierte Hemmung von Faktor-Xa durch Antithrombin-III, herangezogen werden.
Dieser Nachteil der Therapie mit Heparinoiden ist besonders dann sehr unbefriedigend und hinderlich, wenn andere Effekte der Heparinoide als ihre gerinnungshemmende Wirkung im Zentrum des Interesses stehen oder wenn bei lokal begrenzter Therapie die genaue Konzentration des Heparinoids am Wirkort, seine Verweildauer und sein Verteilungsverhalten bekannt sein müssen, wie dies z.B. bei der intraartikulären Injektion zur Therapie von Osteoarthrose oder für die Anwendung bei Schlaganfall, Angina pectoris, Embolien oder in der Tumortherapie erforderlich ist.

Andererseits liegt ein grundsätzliches Problem aller bekannten Therapien der Osteoarthrose in der schwierigen Diagnose des Behandlungserfolges im betroffenen Knorpel- und Knochengewebe.

Es wurde nun gefunden, dass die erfindungsgemäßen Heparinoid-Derivate die genannten Nachteile beheben können.

Die erfindungsgemäßen Heparinoid-Derivate zeichnen sich dadurch aus, dass sie
- als starke Inhibitoren auf die Aktivität von Aggrecanase, hADAMTS1 und Gelatinase A (MMP-2) wirken und deshalb zur Therapie der Osteoarthrose geeignet sind,
- als gerinnungshemmende Arzneistoffe eine vergleichbare anti-thrombotische Wirkung zeigen wie Heparin oder Enoxaparin,
- jedoch zugleich, im Gegensatz zu Heparinoiden, am Wirkort durch kernspintomographische Bildgebungsverfahren (MRI) direkt beobachtbar sind, so dass die lokale Konzentration nach der Applikation überwacht und das Verteilungsverhalten des Arzneimittels im Patienten verfolgt werden kann. Die erfindungsgemäßen Heparinoid-Derivate sind aus diesem Grund auch besonders für die Anwendung bei Schlaganfall, bei Angina pectoris, bei Embolien und in der Tumortherapie geeignet. Weiterhin eignen sie sich jedoch auch hervorragend, um bei Osteoarthrose aus dem Eindringverhalten im Knorpel während der Therapie diagnostische Rückschlüsse auf den Zustand des erkrankten Bindegewebes zu ziehen.

Die Erfindung betrifft daher Derivate eines Heparinoids,
die ein Heparinoid aus der Reihe Enoxaparin, Nadroparin, Fraxiparin, Dalteparin, Fragmin, Bemiparin, Tinzaparin oder Ardeparin,
einen Chelatbildner, der kovalent an dieses Heparinoid gebunden ist, aus der Reihe Diethylentriamin-N,N,N',N",N"-pentaessigsäuredianhydrid, 1,2-Bis(2-aminoethoxyethan)-N,N,N',N'-tetraessigsäure, Ethylendiamin-N,N,N',N'-tetraessigsäure, 1,4,7,10-Tetraazacyclododecan-1,4,7,10-tetraessigsäure, 1,4,7,10-Tetraazacyclododecan-1,4,7-triessigsäure, Nitrilotriessigsäure, Triethylentetraminhexaessigsäure, 4-Carboxy-5,8,11-tris(carboxymethyl)-1-phenyl-2-oxa-5,8,11-triazatridecan-13-oicsäure oder N,N'-bis(-Pyridoxal-5-phosphat)-ethylendiamin-N,N'-diessigsäure und
ein paramagnetisches Metallkation aus der Reihe der Übergangsmetalle Sc, Ti, V, Cr, Mn, Fe, Co, Ni, Cu, Mo, Ru oder der Lanthaniden La, Ce, Pr, Nd, Pm, Sm, Eu, Gd, Tb, Dy, Ho, Er, Tm, Yb enthalten.

Bevorzugtes Heparinoid ist beispielsweise Enoxaparin.

Bevorzugt werden zur chemischen Modifizierung Anhydride der Chelatbildner eingesetzt, besonders bevorzugt Diethylentriamin- N,N,N',N",N"-pentaessigsäure-dianhydrid (DTPA-anhydrid).

Als paramagnetisches Metallkation besonders bevorzugt ist Gd³⁺, eingesetzt in Form seiner Salze Gadolinium(III)-chlorid-hexahydrat oder Gadolinium(III)-acetat-hydrat.

Ein weiterer Gegenstand der Erfindung sind Heparinoid-Derivate, worin der Gehalt an Übergangselement oder Lanthanid von 1 Mol pro Mol Heparinoid bis zur maximal möglichen Derivatisierung des Heparinoids reichen kann, bevorzugt von 1 Mol bis 20 Mol pro Mol Enoxaparin.

Die Erfindung betrifft auch ein Verfahren zur Herstellung der erfindungsgemäßen Heparinoid-Derivate, das dadurch gekennzeichnet ist, dass man das Heparinoid mit einem aktivierten Chelatbildner zu einem Heparinoidchelat umsetzt und anschließend das Übergangselement oder Lanthanid zufügt.

Bei der Herstellung der erfindungsgemäßen Heparinoid-Derivate geht man beispielsweise so vor, dass zunächst das Heparinoid in einem Puffer gelöst wird. Geeignete Puffer haben einen pH-Wert von 6,0 bis 10,0, bevorzugt pH 8,8. Die Konzentration der Puffer beträgt von 0,01 bis 0,5 molar, bevorzugt 0,1 molar. Geeignete Puffer sind beispielsweise Carbonatpuffer, Boratpuffer oder biologische Puffer auf Sulfonsäurebasis, bevorzugt HEPES (N-(2-Hydroxyethyl)piperazin-N'-(2-ethansulfonsäure)). Danach wird der aktivierte Chelatbildner zugefügt, beispielsweise DTPA-anhydrid. Der aktivierte Chelatbildner kann in fester Form oder als Lösung zugegeben werden.
Die Reaktion erfolgt bei einer Temperatur von 8 °C bis 37 °C, bevorzugt 24 °C.
Der pH-Wert wird während der Reaktion bevorzugt konstant gehalten.

Das Verhältnis von aktivierten Chelatbildner zu Heparinoid beträgt von 1 zu 1 bis 50 zu 1, bevorzugt von 1,5 zu 1 bis 15 zu 1 bezogen auf das Molgewicht.

Danach wird, bevorzugt ohne weitere Reinigung des Heparinoidchelats, das Übergangselement oder Lanthanid zugefügt.
Die Komplexbildung erfolgt bei einer Temperatur von 0 °C bis 37°C, bevorzugt 4 °C.
Der pH-Wert wird auf einen schwach sauren Wert umgestellt und anschließend bevorzugt konstant so belassen. Der pH-Wert beträgt 6,8 bis 5, bevorzugt pH 6,5.

Das Verhältnis von Heparinoidchelat zu dem Übergangselement oder Lanthanid beträgt von 1 zu 1 bis 1 zu 50, bevorzugt von 1 zu 1,5 bis 1 zu 15 bezogen auf das Molgewicht.

Das erhaltene erfindungsgemäße Heparinoid-Derivat kann je nach vorgesehener Verwendung noch weiter gereinigt werden. Beispielsweise kann durch Dialyse oder Gelfiltration das Salz entfernt werden. Danach kann das erhaltene Produkt gefriergetrocknet werden.

Enoxaparin und physiologisch verträgliche Salze von Enoxaparin sind bekannt und lassen sich beispielsweise wie in US 5,389,618 beschrieben herstellen. Es sind Gemische sulfatierter Polysaccharide mit der Grundstruktur der das Heparin bildenden Polysaccharide, die dadurch gekennzeichnet sind, dass sie ein mittleres Molekulargewicht von etwa 4500 Dalton besitzen, das niedriger ist als dasjenige des Heparins, dass sie zwischen 9 % und 20 % Ketten mit einem Molekulargewicht von weniger als 2000 Dalton und nur zwischen 5 % und 20 % Ketten mit einem Molekulargewicht von mehr als 8000 Dalton enthalten und dass in ihnen das Verhältnis von gewichtsmittlerem Molekulargewicht zu zahlenmittlerem Molekulargewicht zwischen 1,3 und 1,6 liegt.

Die Erfindung betrifft auch Arzneimittel, gekennzeichnet durch einen wirksamen Gehalt von mindestens einem Heparinoid-Derivat und/oder einem physiologisch verträglichen Salz des Heparinoid-Derivats zusammen mit einem pharmazeutisch geeigneten und physiologisch verträglichen Trägerstoff, Zusatzstoff und/oder anderen Wirk- und Hilfsstoffen.

Die Herstellung physiologisch verträglicher Salze aus zur Salzbildung befähigten Heparinoid-Derivaten erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Omithin oder Arginin, stabile Alkali-, Erdalkali- oder gegebenenfalls substituierte Ammoniumsalze. Sofern die Heparinoid-Derivate basische Gruppen aufweisen, lassen sich mit starken Säuren auch stabile Säureadditionssalze herstellen. Hierfür kommen sowohl anorganische als auch organische Säuren wie beispielsweise Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Als häufig verwendete Hilfsstoffe seien Laktose, Mannit und andere Zucker, Magnesiumcarbonat, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie steriles Wasser, Dimethylsulfoxid (DMSO) und ein- oder mehrwertige Alkohole wie beispielsweise Glycerin, genannt.

Aufgrund der pharmakologischen Eigenschaften eignen sich die erfindungsgemäßen Heparinoid-Derivate zur Prophylaxe und Therapie all solcher Erkrankungen, in deren Verlauf eine verstärkte katabole Aktivität von Proteinasen wie Metalloproteinasen eine maßgebliche Rolle spielt.

Dies ist beispielsweise der Fall bei degenerativen Gelenkerkrankungen wie Osteoarthrosen, Spondylosen, Knorpelschwund nach Gelenktrauma oder längerer Gelenksruhigstellung in der Folge von Meniskus- oder Patellaverletzungen oder Bänderrissen; femer auch bei Erkrankungen des Bindegewebes wie Kollagenosen, Periodontalerkrankungen, Wundheilungsstörungen und chronischen Erkrankungen des Bewegungsapparates wie entzündlichen, immunologisch oder stoffwechselbedingten akuten und chronischen Arthritiden, Arthropathien, Myalgien und Störungen des Knochenstoffwechsels

Ebenso können die erfindungsgemäßen Heparinoid-Derivate vorteilhaft als anti-thrombotische Mittel eingesetzt werden. Insbesondere sind sie bei der Verhütung von venösen Thrombosen in Situationen mit Risiken anwendbar. Dies gilt auch für Situationen mit lang anhaltendem Risiko. Insbesondere ermöglichen es die erfindungsgemäßen Heparinoid-Derivate, bei festgelegten Dosen die Risiken thrombotischer Unfälle in der orthopädischen Chirurgie zu verringern.

Eine vorteilhafte therapeutische Verwendung der erfindungsgemäßen Heparinoid-Derivate beruht auf der Verhütung arterieller, thrombotischer Unfälle, insbesondere im Fall eines Herzinfarkts, bei instabiler Angina pectoris oder wiederkehrender Angina. Eine weitere interessante Anwendung der erfindungsgemäßen Heparinoid-Derivate beruht auf der Möglichkeit, sie zur Verhinderung von venösen Thrombosen bei chirurgischen Patienten im post-operativen Bereich zu verwenden. Diese Anwendung ist überaus vorteilhaft, da sie es erlaubt, während der Operation die Risiken einer Hämorrhagie zu vermeiden. Gleichermaßen vorteilhaft ist die Anwendung der erfindungsgemäßen Heparinoid-Derivate nach der Angiographie und in der Stenose- und Restenose-Therapie.

Weitere mögliche Applikationen der erfindungsgemäßen Heparinoid-Derivate betreffen vorteilhafte Effekte in der Tumor- und Metastasetherapie der Onkologie (antiproliferative Wirkung), bei der Therapie entzündlicher Erkrankungen (antiinflammatorische Wirkung), bei Erkrankungen des Zentralen Nervensystems (ZNS), wie auch bei Transplantationen. Ebenso ist die Applikation möglich bei Ischämien bei Myokardialen und Cerebralen Infarkten (Reduktion der Infarktgröße), bei Asthma (Wirkung auf Tryptase) oder der Angiogenese (fördernde Wirkung der FGF vermittelten Zellproliferation).

Die Applikation der erfindungsgemäßen Heparinoid-Derivate erfolgt im allgemeinen parenteral. Sie kann durch subkutane, intraartikuläre, intraperitoneale oder intravenöse Injektion erfolgen. Die rektale, orale, inhalative oder transdermale Applikation ist ebenfalls möglich. Bei Osteoarthrose bevorzugt ist die intraartikuläre Injektion.

Vorzugsweise werden die pharmazeutischen Präparate in Dosierungseinheiten hergestellt und verabreicht, wobei jede Einheit als aktiven Bestandteil eine bestimmte Dosis des erfindungsgemäßen Heparinoid-Derivats enthält.
Bei Injektionslösungen in Ampullenform kann diese Dosis von etwa 0,5 µg bis zu etwa 200 mg betragen, für die systemische Gabe vorzugsweise von etwa 10 mg bis 80 mg, zur lokalen Applikation vorzugsweise 1 µg bis 10 mg.

Gegenstand der Erfindung ist auch die Verwendung der erfindungsgemäßen Heparinoid-Derivate zur Überwachung und Diagnose des Krankheitsverlaufes bei Erkrankungen, an deren Verlauf eine verstärkte Aktivität von Metalloproteinasen beteiligt ist.

Gegenstand der Erfindung ist auch die Verwendung der erfindungsgemäßen Heparinoid-Derivate zur Herstellung eines Diagnosetestsystems.
Ein weiterer Gegenstand der Erfindung ist die Verwendung eines solchen Diagnosetestsystems zur Überwachung des Behandlungserfolges und funktionalen Charakterisierung bei Erkrankungen.

### Beispiel 1

### Herstellung von Enoxaparin

### 1. Veresterung

Zu einer Lösung von 15 g Benzethoniumheparinat in 75 ml Methylenchlorid gab man 15 ml Benzylchlorid. Die Lösung wurde auf eine Temperatur von 35 °C erhitzt, welche 25 Stunden beibehalten wurde. Man gab dann 90 ml einer 10%igen Natriumacetatlösung in Methanol zu, filtrierte, wusch mit Methanol und trocknete. Man erhielt so 6,5 g Heparinbenzylester in Form des Natriumsalzes, dessen Veresterungsgrad, bestimmt wie vorstehend angegeben, 13,3 % betrug.

### 2. Depolymerisation

10 g des vorstehend erhalten Heparinbenzylester in Form des Natriumsalzes wurden in 250 ml Wasser gelöst. Zu dieser auf 62 °C erhitzten Lösung gab man 0,9 g Natronlauge. Die Temperatur wurde 1 Stunde und 30 Minuten bei 62 °C gehalten. Danach wurde das Reaktionsgemisch auf 20 °C abgekühlt und durch Zugabe von verdünnter Chlorwasserstoffsäure neutralisiert. Man stellte dann die Konzentration des Reaktionsmilieus auf 10 % an Natriumchlorid ein. Das Produkt wurde schließlich in 750 ml Methanol ausgefällt, filtriert und getrocknet. Man erhielt so ein Heparin mit den folgenden strukturellen Merkmalen:
mittleres Molekulargewicht ca. 4500 Dalton
molekulare Verteilung:
   20 % Ketten mit einem Molekulargewicht von geringer als 2000 Dalton
   5,5 % Ketten mit einem Molekulargewicht von höher als 8000 Dalton
Dispersion: d = 1,39

### Beispiel 2

### Herstellung eines Enoxaparinderivats (EN-15)

100 mg Enoxaparin (Feststoff, hergestellt wie in Beispiel 1 beschrieben) wurden in 5 ml eines 0,1-molaren HEPES-Puffers pH 8,8 gelöst. Unter Rühren bei 24 °C tropfte man eine vorbereitete Suspension hinzu, die aus 119 mg Diethylentriamin-N,N,N',N",N"-pentaessigsäuredianhydrid (DTPA-anhydrid) und 0,34 ml Dimethylsulfoxid zubereitet wurde, entsprechend einem 15-fachen molaren Überschuss an Reagenz über die vorgelegte Menge Enoxaparin.
Während der Zugabe wurde der pH-Wert überwacht und durch Nachdosieren von 1-molarer Natronlauge bei pH8,8 gehalten. Der Reaktionsansatz wurde bei Raumtemperatur 30 Minuten lang intensiv gerührt, der pH-Wert wurde hierbei, falls erforderlich, weiterhin durch Zugabe von 1-molarer Natronlauge konstant gehalten. Anschließend wurden dem Ansatz 123,8 mg festes Gadolinium(III)-chlorid-hexahydrat zugegeben, entsprechend einem ebenfalls 15-fachen molaren Überschuss über das enthaltene Enoxaparin. Durch Titration mit 1 N Salzsäure wurde auf pH 6,5 eingestellt. Der Reaktionsansatz wurde 24 Stunden bei 4 °C weiter gerührt.

Die modifizierte Enoxaparinfraktion wurde durch Gelfiltration an Sephadex G-25® entsalzt und von dem nicht umgesetzten Reagenz abgetrennt. Hierzu können auch handelsübliche Pharmacia PD-10® Fertigsäulen entsprechend der Beschreibung des Herstellers eingesetzt werden.
Das modifizierte Enoxaparin wurde gefriergetrocknet.

Es wurde 91 mg eines modifizierten Enoxaparins erhalten. Der Faktor-Xa-Hemmtest zeigte eine mit dem ursprünglichen Enoxaparin vergleichbare Inhibitorstärke. Die Analyse des Gadolinium-Einbaus mittels induktiv gekoppelter Plasma-Atom-Emissionsspektro-metrie ergab einen durchschnittlichen Gehalt von 2 Mol Gadolinium pro Mol Enoxaparin.

### Beispiel 3

### Herstellung eines Enoxaparinderivats (EN-15A)

2g Enoxaparin (Feststoff) wurden in 100 ml eines 0,1-molaren HEPES-Puffers pH 8,8 gelöst. Unter Rühren bei 24 °C tropfte man eine vorbereitete Suspension zu, die aus 2,38 g Bis-(2-aminoethyl)-amin-N,N,N',N",N"-pentaessigsäuredianhydrid (DTPA-anhydrid) und 6,8 ml Dimethylsulfoxid zubereitet wurde, entsprechend einem 15-fachen molaren Überschuss an Reagenz über die vorgelegte Menge Enoxaparin. Während der Zugabe wurde der pH-Wert überwacht und durch Nachdosieren von 1-molarer Natronlauge bei pH 8,8 gehalten. Der Reaktionsansatz wurde bei Raumtemperatur 30 Minuten lang intensiv gerührt, der pH-Wert wurde hierbei, falls erforderlich, weiterhin durch Zugabe von 1-molarer Natronlauge konstant gehalten. Anschließend wurden in den Ansatz 2,48 g festes Gadolinium(III)-chlorid-hexahydrat eingerührt, entsprechend einem ebenfalls 15-fachen molaren Überschuss über das enthaltene Enoxaparin. Durch Titration mit 1-molarer Salzsäure wurde auf pH 6,5 eingestellt. Der Reaktionsansatz wurde 24 Stunden bei 4 °C weiter gerührt.
Die modifizierte Enoxaparinfraktion wurde nun durch 24-stündige Dialyse gegen insgesamt 4 Volumina von jeweils 51 Wasser in einem handelsüblichen Dialyseschlauch (Trenngrenze Molekulargewicht 1000) entsalzt und von dem nicht umgesetzten Reagenz befreit. Hierbei wurde das vorgelegte Wasser nach 1 Stunde, 3 Stunden und 16 Stunden jeweils durch frisches Wasser ersetzt. Der Schlauchinhalt wurde anschließend gefriergetrocknet.
Es wurde 1,77 g eines modifizierten Enoxaparins erhalten.
Vorzugsweise wurde das Produkt durch Gelfiltration an Sephadex G-25® unter Verwendung von pyrogenfreiem Wasser nochmals gereinigt.
Der Faktor-Xa-Hemmtest zeigte eine mit dem ursprünglichen Enoxaparin vergleichbare Inhibitorstärke.
Die Analyse des Gadolinium-Einbaus mittels induktiv gekoppelter Plasma-Atom-Emissionsspektrometrie ergab einen Gehalt von 5 Mol Gadolinium pro Mol Enoxaparin.

### Beispiel 3a

### Herstellung eines Enoxaparinderivats (EN-15B)

2 g Enoxaparin (Feststoff) wurden in 100 ml eines 0,1-molaren HEPES-Puffers pH 8,8 gelöst. Unter Rühren bei 24 °C tropfte man eine vorbereitete Suspension zu, die aus 2,38 g Bis-(2-aminoethyl)-amin-N,N,N',N",N"-pentaessigsäuredianhydrid (DTPA-anhydrid) und 6.8 ml Dimethylsulfoxid vorbereitet wurde, entsprechend einem 15-fachen molaren Überschuß an Reagenz über die vorgelegte Menge Enoxaparin. Während der Zugabe wurde der pH-Wert überwacht und durch Nachdosieren von 1-molarer Natronlauge bei pH 8,8 gehalten. Der Reaktionsansatz wurde bei Raumtemperatur 30 Minuten lang intensiv gerührt, der pH-Wert wurde hierbei, falls erforderlich, weiterhin durch Zugabe von 1-molarer Natronlauge konstant gehalten. Anschließend wurden in den Ansatz 2,48 g festes Gadolinium(III)-chlorid-hexahydrat eingerührt, entsprechend einem ebenfalls 15-fachen molaren Überschuß über das enthaltene Enoxaparin.
Durch Titration mit 1 N Salzsäure wurde auf pH 6,5 eingestellt. Der Reaktionsansatz wurde 24 Stunden bei 4 °C weiter gerührt.
Nach Ablauf dieser Zeit gab man 0,4 ml Ethanolamin hinzu und ließ bei Raumtemperatur noch 30 Minuten lang rühren.
Schließlich wurde durch Zugabe von Salzsäure auf pH 7,0 eingestellt, und der Reaktionsansatz wurde durch Verdünnung mit dem 4-fachen Volumen an Methanol präzipitiert. Der Niederschlag wurde über ein Filter abgesaugt und mit 30 ml reinem Wasser sehr konzentriert wieder gelöst.
Das modifizierte Enoxaparin wurde durch 24-stündige Dialyse gegen insgesamt 4 Volumina von jeweils 0,5 l Wasser in einem handelsüblichen Dialyseschlauch (Trenngrenze Molekulargewicht 1000) entsalzt und von dem nicht umgesetzten Reagenz befreit. Hierbei wurde das vorgelegte Wasser nach 1 Stunde, 3 Stunden und 16 Stunden jeweils durch frisches Wasser ersetzt. Der Schlauchinhalt wurde anschließend gefriergetrocknet.
Zeitsparend kann die Entsalzung für kleinere Volumina auch mit einer handelsüblichen Gelfiltrationssäule durchgeführt werden (z.B. Pharmacia HiPrep™ Desalting).
Die entsalzte Fraktion wird dann gleichermaßen gefriergetrocknet.
Es wurden 1,86 g eines modifizierten Enoxaparins erhalten. Der Faktor-Xa-Hemmtest zeigte eine im Vergleich zum ursprünglichen Enoxaparin ungeschwächte Inhibitorstärke. Die Analyse des Gadolinium-Einbaus mittels induktiv gekoppelter Plasma-Atom-Emissionsspektrometrie ergab einen Gehalt von 2,7 Mol Gadolinium pro Mol Enoxaparin.

### Beispiel 4

Gemäß Beispiel 2 wurden auch Enoxaparinderivate mit dem 3-fachen, dem 8-fachen und dem 50-fachen Überschuss an DTPA-anhydrid und Gadolinium(III)-chlorid-hexahydrat hergestellt. Die Verbindungen werden im folgenden mit EN 3, EN8 und EN-50 abgekürzt bezeichnet.
Ebenso wurde gemäß Beispiel 2 auch ein Präparat hergestellt, welches mit dem 50-fachen Überschuß an DTPA-anhydrid umgesetzt, anschließend jedoch nicht mit Gadoliniumionen beladen wurde. Dieses Produkt, im folgenden als EN-50Z bezeichnet, entspricht einem Enoxaparinderivat mit erhöhter anionischer Ladung und entsprechend modulierten pharmakologischen Eigenschaften, welches aber insbesondere auch deswegen von Interesse ist, weil es als chelatisierende Vorstufe auf einfache Weise auch zur Beladung mit anderen, z.B. auch radioaktiven, Kationen benützt werden kann.

### Beispiel 5

### Herstellung eines Heparinderivats (HE-15B)

2 g handelsübliches Heparin-Natrium (Feststoff; Sigma H4784) wurden in 100 ml eines 0,1-molaren HEPES-Puffers pH 8,8 gelöst. Unter Rühren bei 24 °C tropfte man eine vorbereitete Suspension zu, die aus 1,19 g Bis-(2-aminoethyl)-amin-N,N,N',N",N"-pentaessigsäuredianhydrid (DTPA-anhydrid) und 3,4 ml Dimethylsulfoxid vorbereitet wurde.
Während der Zugabe wurde der pH-Wert überwacht und durch Nachdosieren von 1-molarer Natronlauge bei pH 8,8 gehalten. Der Reaktionsansatz wurde bei Raumtemperatur 30 Minuten lang intensiv gerührt, der pH-Wert wurde hierbei, falls erforderlich, weiterhin durch Zugabe von 1-molarer Natronlauge konstant gehalten. Anschließend wurden in den Ansatz 1.24 g festes Gadolinium(III)-chlorid-hexahydrat eingerührt.
Durch Titration mit 1 N Salzsäure wurde auf pH 6,5 eingestellt. Der Reaktionsansatz wird 24 Stunden bei 4 °C weiter gerührt.
Nach Ablauf dieser Zeit gab man 0,2 ml Ethanolamin hinzu und ließ bei Raumtemperatur noch 30 Minuten lang rühren.
Schließlich wurde durch Zugabe von Salzsäure auf pH 7,0 eingestellt, und der Reaktionsansatz wurde durch Verdünnung mit dem 4-fachen Volumen an Methanol präzipitiert. Der Niederschlag wurde über ein Filter abgesaugt und mit 30 ml reinem Wasser sehr konzentriert wieder gelöst.
Das modifizierte Heparin wurde nun durch 24-stündige Dialyse gegen insgesamt 4 Volumina von jeweils 0,5 L Wasser in einem handelsüblichen Dialyseschlauch (Trenngrenze Molekulargewicht 1000) entsalzt und von dem nicht umgesetzten Reagenz befreit. Hierbei wurde das vorgelegte Wasser nach 1 Stunde, 3 Stunden und 16 Stunden jeweils durch frisches Wasser ersetzt. Der Schlauchinhalt wurde anschließend gefriergetrocknet.
Zeitsparend kann die Entsalzung für kleinere Volumina auch mit einer handelsüblichen Gelfiltrationssäule durchgeführt werden (z.B. Pharmacia HiPrep™ Desalting).
Die entsalzte Fraktion wird dann gleichermaßen gefriergetrocknet.
Es wurden 1,65 g eines modifizierten Heparins erhalten. Der Faktor-Xa-Hemmtest zeigte eine im Vergleich zum ursprünglichen Heparin ungeschwächte Inhibitorstärke. Die Analyse des Gadolinium-Einbaus mittels induktiv gekoppelter Plasma-Atom-Emissionsspektrometrie ergab einen Gehalt von 1,9 Mol Gadolinium pro Mol Heparin.

### Beispiel 6

### Testsystem zur Untersuchung der Heparinoid-abhängigen Hemmung von Faktor Xa

### Testprinzip:

Wird eine Testprobe, die ein Heparinoid enthält, mit Antithrombin III und einem Überschuss an Faktor Xa versetzt, so inaktiviert das in der Testprobe mit Antithrombin III zu einem Komplex gebundene Heparinoid den Faktor Xa. Die Restaktivität von Faktor Xa kann mit einem synthetischen chromogenen Substrat gemessen werden. Hierbei wird durch enzymatische Spaltung para-Nitroanilin aus dem Substrat freigesetzt, welches photometrisch durch Messung der Extinktionsänderung pro Zeiteinheit bei einer Wellenlänge von 405 nm detektiert werden kann. Die Menge des freigesetzten para-Nitroanilins ist umgekehrt proportional zur Konzentration des Heparinoids in der Testprobe (Teien M.L. et al., Thromb. Res. 8 (3), 413-6 [1976]). Mit abgestuften Konzentrationen des Heparinoids im untersuchten Medium wird eine Eichreihe erstellt (Extinktionsänderung pro Zeiteinheit in Abhängigkeit von der Konzentration). Aus der Extinktionsänderung einer Testprobe pro Zeiteinheit lässt sich im Vergleich die Konzentration des Heparinoids ermitteln.

### Testdurchführung:

Zur Erstellung der Eichgeraden wird vorzugsweise ein Konzentrationsbereich von 0,5 µg/ml bis 3 µg/ml des Heparinoids verwendet.
Die Proben dieser Konzentrationsreihe werden 1:10 mit 0,046 M Tris-Puffer pH 8,4 , der 0,15 M NaCl, 0,007 M EDTA, 0,1% Tween 80 und 0,12 IU humanes Antithrombin III enthält, verdünnt. Je 50 µl der verdünnten Proben werden mit 50 µl bovinem Faktor Xa (13,6 U/ml) 80 Sekunden lang bei 37 °C inkubiert. Anschließend werden 50 µl 1,1 mM chromogenes Substrat S-2765 zugefügt. Die Extinktionsänderung pro Minute wird photometrisch bei einer Wellenlänge von 405nm gemessen.
Nach dem gleichen Muster wird mit jeweils 50 µl der geeignet vorverdünnten Testproben verfahren.

Tabelle 1 zeigt die Ergebnisse:

**Tabelle 1:**

| Konzentration (µg/ml)* | Enoxaparin | EN-3 | EN-8 | EN-15 | EN-50 | EN-50Z |
|---|---|---|---|---|---|---|
| 0,3 | 0,473 | 0,431 | 0,463 | 0,406 | 0,362 | 0,439 |
| 0,25 | 0,545 | 0,491 | 0,509 | 0,486 | 0,455 | 0,484 |
| 0,2 | 0,607 | 0,544 | 0,579 | 0,577 | 0,519 | 0,558 |
| 0,15 | 0,671 | 0,659 | 0,656 | 0,63 | 0,602 | 0,643 |
| 0,1 | 0,728 | 0,711 | 0,737 | 0,71 | 0,692 | 0,726 |
| 0,05 | 0,806 | 0,794 | 0,808 | 0,786 | 0,793 | 0,794 |
| 0 | 0,884 | 0,897 | 0,897 | 0,896 | 0,907 | 0,910 |

### Beispiel 7

### Aggrecanase Testsystem

Der Test wird im 96-well-Mikrotiterplattenformat durchgeführt.
Zur Vorbereitung wird eine Verdünnungsreihe des gelabelten Enoxaparins in reinem Wasser angelegt.

### Verdauungen:

Pro well wird eine vorgegebene Menge an Synovialflüssigkeit oder Aggrecanase-Aktivität, welche unter den Testbedingungen eine Extinktion von 1.0 bis 1.4 bei 405nm bewirkt, mit 3 µl der jeweiligen Verdünnungsstufe des gelabelten Enoxaparins versetzt, mit Dulbecco's Modified Eagle Medium (DMEM) auf ein Endvolumen von 300µl aufgefüllt und 1 Stunde lang bei 37°C im CO₂-Zellkulturbrutschrank inkubiert. Anschließend werden pro weil 5 µl einer Lösung von 1 µg/µl Agg1mut-Substrat (nach: Bartnik E. et al., EP 785274 (1997); Substrat in DMEM) hinzugegeben, und der Ansatz wird 4 Stunden lang bei 37°C im CO₂-Brutschrank verdaut.

### Vorbereitung der Testplatte:

lm ersten Schritt wird pro well 1 Stunde lang bei Raumtemperatur mit 100□l einer Lösung von handelsüblichem anti-Maus-Immunglobulin-G (aus Ziege; 5µg/ml in physiologischem Phosphatpuffer pH 7.4 [PBS-Puffer]) beschichtet. Nach Waschen der Platte mit PBS-Puffer unter Zusatz von 0.1 % Tween-20 (im Folgenden Waschpuffer genannt) wird pro well 1 Stunde lang bei Raumtemperatur mit 100 µl einer Lösung von 5% Rinderserumalbumin in PBS-Puffer unter Zusatz von 0.05% Tween-20 geblockt. Nach erneutem Waschen mit Waschpuffer wird pro well 1 Stunde lang bei Raumtemperatur mit 100 µl einer 1:1000 verdünnten Lösung von BC-3-Antikörper in PBS-Puffer mit 0.05% Tween-20 und 0.5% Rinderserumalbumin inkubiert;
dieser Antikörper erkennt Aggrecanase-typische Spaltfragmente (Hughes C.E. et al., Biochem. J. (1995), 305 (3), 799-804).

### Testdurchführung:

Nach Waschen der Testplatte mit Waschpuffer wird der komplette Ansatz aus der vorher
durchgeführten Verdauung weil für well auf die Testplatte übertragen und 1 Stunde lang bei Raumtemperatur inkubiert. Nach Waschen der Platte mit Waschpuffer folgt die Zugabe von 100 µl des zweiten Antikörpers (goat anti-human IgG, peroxidaselabeled, 1:1000 in 0,5% BSA/PBS-Puffer/0.05% Tween-20), mit dem wiederum 1 Stunde lang bei Raumtemperatur inkubiert wird.
Nach erneutem Waschen mit Waschpuffer erfolgt die Farbentwicklung durch Zugabe von 100 µl ABTS-Substratlösung (2 mg/ml 2,2'-Azinobis(3-ethylbenzthiazolin-sulfonsäure in 40 mM Natriumcitrat mit 60 mM di-Natriumhydrogenphosphat, mit Essigsäure auf pH 4,4 eingestellt; unmittelbar vor der Messung mit 0,25ml 35% Wasserstoffperoxid pro ml versetzt). Die Messung erfolgt im Schüttelmodus bei 405 nm gegen ein Referenzfilter (620 nm) mit automatischen Ablesungen in 5-Sekunden-Intervallen. Der Test wird abgebrochen, sobald eine maximale Extinktion (405 nm) im Bereich von 1,0 bis 1,4 erreicht ist.

**Tabelle 2: (Substratumsatz in % im Vergleich zum Umsatz der ungehemmten Aggrecanase)**

| Konzentration (µg/ml) | Enoxaparin | EN-3 | EN-8 | EN-15 | EN-50 | EN-50Z | HE-15B |
|---|---|---|---|---|---|---|---|
| 100 | 52,1 | 31,2 | 26,6 | 32,2 | 38,3 | 8,9 | 23,4 |
| 10 | 42,2 | 20,2 | 20,8 | 28,5 | 47,0 | 15,4 | 16.8 |
| 1 | 53,8 | 36,9 | 43,7 | 37,6 | 29,4 | 36,4 | 12,4 |
| 0,1 | 99,1 | 87,6 | 97,5 | 89,5 | 88,4 | 72,9 | 67,7 |
| 0,01 | 116,4 | 113,2 | 109,8 | 106,0 | 108,6 | 89.9 | 92,1 |
| 0,001 | 118,0 | 115,3 | 110,0 | 109,5 | 111,2 | 95.8 | 95,0 |
| 0,0001 | 107,8 | 107,2 | 106,7 | 101,0 | 102,3 | 98.2 | 99,3 |

### Beispiel 8

### Kernspintomographisches Bildgebungsexperiment

Die in den Beispielen 3 und 4 beschriebenen erfindungsgemäßen Heparinoid-Derivate EN-3, EN-8, EN-15 und EN-50 werden in destilliertem Wasser in den Konzentrationen 0,01, 0,1, 1,0 und 10,0 mM aufgelöst und in 0,5 Milliliter fassende Eppendorf-Röhrchen abgefüllt. Jedes Röhrchen wird in ein größeres, 1,5 Milliliter fassendes und mit destilliertem Wasser gefülltes Eppendorf-Röhrchen eingestellt. Diese Röhrchen werden in einem Kunststoffgestell angeordnet und in einem Kernspintomographen der Firma Bruker Medical GmbH, Ettlingen, bei einer Magnetfeldstärke von 7 Tesla abgebildet.
Zur Beschreibung der Wirkung der erfindungsgemäßen Heparinoid-Derivate auf die Relaxationszeiten der Wasserprotonen in den Lösungen werden MR-Bilder mit unterschiedlichem Kontrastverhalten unter Verwendung der von der Firma Bruker Medical GmbH entwickelten Software "Paravision"® gemessen. Um alle Röhrchen gleichzeitig im Bild zu sehen, wird eine koronale (= horizontale) Schnittebene mit 2 mm Schichtdicke gewählt.

In T1-gewichteten Spin-Echo-Bildern (Echozeit TE=13 msec, Relaxationszeit TR=100 msec, 1 Echo, NA=2, Matrix 256²) zeigen die Röhrchen mit allen Derivaten bei der Konzentration 10 mM Signalauslöschung, bei EN-50 und EN-15 sogar eine Verzerrung der Abbildung auf Grund der örtlichen Beeinträchtigung der Homogenität des Magnetfeldes durch die höhere Gadolinium-Konzentration je Mol Enoxaparin. In den Röhrchen mit EN-50 und EN-15 bei 0,1 mM Konzentration sowie denjenigen mit den Derivaten EN-8 und EN-3 bei 1.0 mM Konzentration erfolgt eine Signalverstärkung bezogen auf das Signal des umgebenden destillierten Wassers um etwa den Faktor 10. Die Signalverstärkung in den restlichen Röhrchen vermindert sich entsprechend der geringeren Konzentration der erfindungsgemäßen Heparinoid-Derivate bzw. der geringeren relativen Gadolinium-Konzentration.

Die Relaxationszeit T2 für die einzelnen Lösungen wird in einem Spin-Echo-Experiment mit 16 einzelnen Echos ermittelt (TE=13 msec, TR=3000 msec, 16 Echos, NA=1,
Matrix 256²). Die Ergebnisse sind in der Tabelle 3 zahlenmäßig zusammengefasst. In allen 10-mM-Lösungen der erfindungsgemäßen Heparinoid-Derivate ist die T2-Zeit derart verkürzt, dass sie nicht mehr zuverlässig ermittelt werden kann. Bezogen auf die unter diesen Messbedingungen ermittelte T2-Zeit von reinem destillierten Wasser (907 msec) wird eine maximale Verkürzung um den Faktor 60 erreicht. Die Verkürzung der T2-Zeit ist proportional zur Konzentration der erfindungsgemäßen Heparinoid-Derivate sowie der individuellen relativen Gadolinium-Konzentration.

### Zusammenfassung:

Die MRI-Untersuchung zeigt, dass die erfindungsgemäßen Heparinoid-Derivate in Abhängigkeit von ihrer Konzentration und in Abhängigkeit vom Molverhältnis Gadolinium zu Enoxaparin die Relaxationszeiten T1, T2 und T2* der Wässerprotonen von Lösungen verkürzen, in denen die erfindungsgemäßen Heparinoid-Derivate enthalten sind, so dass es in T1-gewichteten MRI-Bildern zu einer Erhöhung der Signalintensität oder sogar zu einer partiellen oder totalen Signalauslöschung kommt.

**Tabelle 3: (T2 - Relaxationszeiten)**

| Derivat | 0,01 mM | 0,1 mM | 1,0 mM | 10 mM | Wasser_{dest} |
|---|---|---|---|---|---|
| EN-50 | 201 | 23 | nb* | nb* | 995 |
| EN-15 | 347 | 47 | nb* | nb* | 871 |
| EN-8 | 648 | 124 | 14,4 | nb* | 845 |
| EN-3 | 682 | 124 | 14,3 | nb* | 917 |

| | | | | | |
|---|---|---|---|---|---|
| Gemessene T2-Zeiten (in msec) der Wasserprotonen in Lösungen der erfindungsgemäßen Heparinoid-Derivate in den angegebenen millimolaren Konzentrationen. | | | | | |
| Zur Bestimmung der T2-Zeiten wird eine Spin-Echo-Abbildungssequenz mit 32 einzelnen Echos verwendet. Der Mittelwert für die T2-Relaxation im umgebenden destillierten Wasser beträgt 907 msec. | | | | | |
| (* nb = T2 nicht mehr zuverlässig bestimmbar, da zu kurz) | | | | | |

### Beispiel 9

### Kernspintomographisches Bildgebungsexperiment

Das isolierte Kniegelenk eines Schweins (ca. 40 kg schwer, 4 Monate alt) wird freigelegt. Das Gelenk wird in einem Plastikbehälter so befestigt, dass nur die eine Kondyle in eine Lösung von EN-15A (0.1 mM in physiologischer Kochsalzlösung, Raumtemperatur) eintaucht. Über einen Zeitraum von 14 Stunden wird alle 30 Minuten je eine T1-gewichtete Spin-Echo-Aufnahme mit hoher räumlicher Auflösung (Voxelgröße ca. 140 x 180 µm, Schichtdicke 2 mm) gemessen. Das Schnittbild, etwa sagittal durch den Gelenkkopf und Knochenschaft, zeigt den Bereich des trabekulären Knochens grau bis schwarz umgeben von einer hellgrauen Schicht Knorpel, die eine Dicke von bis zu
7 mm erreicht. Die Grenzflächen Knochen / Knorpel sowie Knorpel / umgebende Lösung sind klar erkennbar.
Einige Stunden nach Eintauchen in die EN-15A-Lösung ändert sich das Aussehen der äußeren Knorpelschicht. Es bilden sich neue laminare Strukturen aus, die in etwa parallel zur Knorpeloberfläche verlaufen: eine erste oberflächennahe dünne (ca. 140 µm tief) hyperintense Schicht, der eine breitere (ca. 500 µm tiefe) hypointense Schicht folgt. In dieser zweiten Schicht findet zunehmend eine Signalauslöschung statt, die sowohl mit einer erhöhten Konzentration an EN-15A wie auch mit einer stark verringerten Beweglichkeit des EN-15A in dieser Knorpelzone erklärt werden kann, da beide Effekte einen so schnellen Signalabfall zu induzieren vermögen, dass kein Signal mehr messbar ist.

Es schließt sich eine dritte hyperintense Schicht an, die ähnlich breit und hell ist wie die erste. Es folgt eine vierte, stark hyperintense Schicht, die etwa doppelt bis dreifach so breit ist wie die zweite. Im Anschluss ist noch eine fünfte Schicht zu erkennen, deren Signalintensität in etwa jener der ersten oder der dritten Schicht entspricht, gleichwohl etwas breiter als diese ist.
Die tiefste Front an EN-15A liegt etwa 1,6 bis 1,7 mm von der Knorpeloberfläche entfernt.

### Zusammenfassung:

Die MRI-Untersuchung zeigt, dass die erfindungsgemäßen Heparinoid-Derivate den nativen intakten Knorpel eines isolierten femoralen Gelenkkopfes vom Schwein durchdringen, wobei die erfindungsgemäßen Heparinoid-Derivate eine Änderung der MR-Signalintensität auf Grund der Beeinflussung der Relaxationszeiten T1, T2 und T2* der Wasserprotonen im Knorpel bewirken; diese Änderung der Relaxationszeiten T1, T2 und T2* der Wasserprotonen wird durch die reduzierte Beweglichkeit der erfindungsgemäßen Heparinoid-Derivate innerhalb des Knorpels verstärkt.

## Patentansprüche

1. Derivate eines Heparinoids,
die ein Heparinoid aus der Reihe Enoxaparin, Nadroparin, Fraxiparin, Dalteparin, Fragmin, Bemiparin, Tinzaparin oder Ardeparin,
einen Chelatbildner, der kovalent an dieses Heparinoid gebunden ist, aus der Reihe Diethylentriamin-N,N,N',N",N"-pentaessigsäuredianhydrid, 1,2-Bis(2-aminoethoxyethan)-N,N,N',N'-tetraessigsäure, Ethylendiamin-N,N,N',N'-tetraessigsäure, 1,4,7,10-Tetraazacyclododecan-1,4,7,10-tetraessigsäure, 1,4,7,10-Tetraazacyclododecan-1,4,7-triessigsäure, Nitrilotriessigsäure, Triethylentetraminhexaessigsäure, 4-Carboxy-5,8,11-tris(carboxymethyl)-1-phenyl-2-oxa-5,8,11-triazatridecan-13-oicsäure oder N,N'-bis(-Pyridoxal-5-phosphat)-ethylendiamin-N,N'-diessigsäure und
ein paramagnetisches Metallkation aus der Reihe der Übergangsmetalle Sc, Ti, V, Cr, Mn, Fe, Co, Ni, Cu, Mo, Ru oder der Lanthaniden La, Ce, Pr, Nd, Pm, Sm, Eu, Gd, Tb, Dy, Ho, Er, Tm, Yb enthalten.

2. Heparinoid-Derivat gemäß Anspruch 1, **dadurch gekennzeichnet, dass** als Heparinoid Enoxaparin eingesetzt wird.

3. Derivate eines Heparinoids gemäß der Ansprüche 1 oder 2, **dadurch gekennzeichnet, dass** das paramagnetische Metallkation Gd³⁺ ist.

4. Heparinoid-Derivat gemäß einem oder mehreren der Ansprüche 1 bis 3, **dadurch gekennzeichnet, dass** der Gehalt an Übergangselement oder Lanthanid von 1 Mol pro Mol bis zur maximal möglichen Derivatisierung des eingesetzten Heparinoids beträgt, bevorzugt von 1 Mol bis 20 Mol pro Mol Enoxaparin.

5. Verfahren zur Herstellung der Heparinoid-Derivate gemäß einem oder mehreren der Ansprüche 1 bis 4, **dadurch gekennzeichnet, dass** man das Heparinoid mit einem aktivierten Chelatbildner zu einem Heparinoidchelat umsetzt und anschließend das Übergangselement oder Lanthanid zufügt.

6. Verfahren gemäß Anspruch 5, **dadurch gekennzeichnet, dass** die aktivierte Form des Chelatbildners gegenüber dem vorgelegten Heparinoid in einem Überschuss von 1 zu 1 bis 50 zu 1 bezogen auf das Molgewicht eingesetzt wird, bevorzugt von 1,5 zu 1 bis 15 zu 1.

7. Verfahren gemäß der Ansprüche 5 oder 6, **dadurch gekennzeichnet, dass** die aktivierte Form des Chelatbildners ein Anhydrid ist wie Diethylentriamin-N,N,N',N",N"-pentaessigsäuredianhydrid.

8. Verfahren gemäß einem oder mehreren der Ansprüche 5 bis 7, **dadurch gekennzeichnet, dass** Gd3+ in Form seiner Salze Gadolinium(III)-chlorid-hexahydrat oder Gadolinium(III)-acetat-hydrat eingesetzt wird.

9. Verfahren gemäß einem oder mehreren der Ansprüche 5 bis 8, **dadurch gekennzeichnet, dass** das Verhältnis von Heparinoid-Chelat zu dem Übergangselement oder Lanthanid bei der Zugabe von 1 zu 1 bis 1 zu 50 bezogen auf das Molgewicht gewählt wird, bevorzugt von 1 zu 1,5 bis 1 zu 15.

10. Arzneimittel, **gekennzeichnet durch** einen wirksamen Gehalt von mindestens einem Heparinoid-Derivat gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

11. Verwendung der Heparinoid-Derivate gemäß einem oder mehreren der Ansprüche 1 bis 4 zur Herstellung eines Arzneimittels zur Prophylaxe und Therapie von Erkrankungen, die durch eine verstärkte katabole Aktivität von Proteinasen **gekennzeichnet** sind wie degenerative Gelenkerkrankungen, Osteoarthrosen, Spondylosen, Knorpelschwund nach Gelenktrauma oder längerer Gelenksruhigstellung in der Folge von Meniskus- oder Patellaverletzungen oder Bänderrissen, Erkrankungen des Bindegewebes wie Kollagenosen oder Periodontalerkrankungen, Wundheilungsstörungen oder chronische Erkrankungen des Bewegungsapparates wie entzündliche, immunologisch oder stoffwechselbedingte akute und chronische Arthritiden, Arthropathien, Myalgien oder Störungen des Knochenstoffwechsels.

12. Verwendung der Heparinoid-Derivate gemäß einem oder mehreren der Ansprüche 1 bis 4 zur Herstellung eines Arzneimittels zur anti-thrombotischen Therapie und Prophylaxe wie der Verhütung von venösen Thrombosen, zur Verhinderung von venösen Thrombosen bei chirurgischen Patienten im post-operativen Bereich, zur Verhütung arterieller, thrombotischer Unfälle, insbesondere im Fall eines Herzinfarkts, bei instabiler Angina pectoris oder wiederkehrender Angina, zur Anwendung nach der Angiographie und in der Stenose- und Restenose-Therapie, zur Tumor- und Metastasetherapie, zur Therapie entzündlicher Erkrankungen, zur Behandlung von Ischämien bei myokardialen oder cerebralen Infarkten, zur Therapie bei Erkrankungen des Zentralen Nervensystems, zur Therapie bei Transplantationen, zur Therapie von Asthma oder zur Therapie der Angiogenese.

13. Verwendung gemäß der Ansprüche 11 und 12, **dadurch gekennzeichnet, dass** die Applikation der Heparinoid-Derivate parenteral wie durch subkutane, intraartikuläre, intraperitoneale oder intravenöse Injektion erfolgt.

14. Verwendung gemäß Anspruch 13, **dadurch gekennzeichnet, dass** die Dosis des Heparinoid-Derivats für die systemische Gabe von etwa 10 mg bis 80 mg und für die lokale Applikation von 1 µg bis 10 mg beträgt.

15. Verwendung der Heparinoid-Derivate gemäß einem oder mehreren der Ansprüche 1 bis 4 zur Überwachung und Diagnose des Krankheitsverlaufes bei Erkrankungen, an deren Verlauf eine verstärkte Aktivität von Metalloproteinasen beteiligt ist.

16. Verwendung der Heparinoid-Derivate gemäß einem oder mehreren der Ansprüche 1 bis 4 zur Herstellung eines Diagnosetestsystems.

17. Verwendung eines Diagnosetestsystems gemäß des Anspruchs 16 zur Überwachung des Behandlungserfolges und funktionalen Charakterisierung bei Erkrankungen.

## Claims

1. A derivative of a heparinoid which comprises a heparinoid from the series enoxaparin, nadroparin, Fraxiparin, dalteparin, Fragmin, bemiparin, tinzaparin or ardeparin, a chelating agent which is covalently bonded to this heparinoid from the series diethylenetriamine-N,N,N',N",N"-pentaacetic acid dianhydride, 1,2-bis(2-aminoethoxyethane)-N,N,N',N'-tetraacetic acid, ethylenediamine-N,N,N',N'-tetraacetic acid, 1,4,7,10-tetraazacyclododecane-1,4,7,10-tetraacetic acid, 1,4,7,10-tetraazacydododecane-1,4,7-triacetic acid, nitrilotriacetic acid, triethylenetetraminehexaacetic acid, 4-carboxy-5,8,11-tris(carboxymethyl)-1-phenyl-2-oxa-5,8,11-triazatridecan-13-oic acid or N,N'-bis(pyridoxal 5-phosphate)-ethylenediamine-N,N'-diacetic acid, and a paramagnetic metal cation from the series of transition metals Sc, Ti, V, Cr; Mn, Fe, Co, Ni, Cu, Mo, Ru or of lanthanides La, Ce, Pr, Nd, Pm, Sm, Eu, Gd, Tb, Dy, Ho, Er, Tm, Yb.

2. A heparinoid derivative as claimed in claim 1, wherein enoxaparin is employed as heparinoid.

3. A derivative of a heparinoid as claimed in claim 1 or 2, wherein the paramagnetic metal cation is Gd³⁺.

4. A heparinoid derivative as claimed in one or more of claims 1 to 3, wherein the content of transition element or lanthanide is from 1 mol per mol up to the maximum possible derivatization of the heparinoid employed, preferably from 1 mol to 20 mol per mol of enoxaparin.

5. A process for preparing the heparinoid derivatives as claimed in one or more of claims 1 to 4, which comprises reacting the heparinoid with an activated chelating agent to give a heparinoid chelate, and subsequently adding the transition element or lanthanide.

6. The process as claimed in claim 5, wherein the activated form of the chelating agent is employed in an excess of from 1:1 to 50:1 in relation to the initial heparinoid, based on the molecular weight, preferably from 1.5:1 to 15:1.

7. The process as claimed in claims 5 or 6, wherein the activated form of the chelating agent is an anhydride such a diethylenetriamine-N,N,N',N",N"-pentaacetic acid dianhydride.

8. The process as claimed in one or more of claims 5 to 7, wherein Gd³⁺ is employed in the form of its salts gadolinium(III) chloride hexahydrate or gadolinium(III) acetate hydrate.

9. The process as claimed in one or more of claims 5 to 8, wherein the chosen ratio of heparinoid chelate to transition element or lanthanide on addition is from 1:1 to 1:50 based on the molecular weight, preferably from 1:1.5 to 1:15.

10. A medicament having an effective content of at least one heparinoid derivative as claimed in one or more of claims 1 to 4 together with a pharmaceutically suitable and physiologically tolerated carrier, additive and/or other active ingredients and excipients.

11. The use of the heparinoid derivatives as claimed in one or more of claims 1 to 4 for producing a medicament for the prophylaxis and therapy of disorders **characterized by** an increased catabolic activity of proteinases, such as degenerative joint disorders, osteoarthroses, spondyloses, chondrolysis after joint trauma or prolonged immobilization of joints following meniscus or patella injuries or torn ligaments, disorders of connective tissue such as collagenoses or periodontal disorders, disturbances of wound healing or chronic disorders of the locomotor system such as inflammatory, immunology- or metabolism-related acute and chronic arthritis, arthropathies, myalgias and disturbances of bone metabolism.

12. The use of the heparinoid derivatives as claimed in one or more of claims 1 to 4 for producing a medicament for antithrombotic therapy and prophylaxis such as the prevention of venous thromboses, for preventing venous thromboses in surgical patients in the postoperative period, for preventing arterial thrombotic events, especially in the case of a myocardial infarction, of unstable angina pectoris or recurrent angina, for use after angiography and in stenosis and restenosis therapy, for tumor and metastasis therapy, for the therapy of inflammatory disorders, for the treatment of ischemias associated with myocardial or cerebral infarctions, for the therapy of disorders of the central nervous system, for therapy associated with transplants, for the therapy of asthma or for the therapy of angiogenesis.

13. The use as claimed in claims 11 and 12, wherein the heparinoid derivatives are administered parenterally such as by subcutaneous, intra-articular, intraperitoneal or intravenous injection.

14. The use as claimed in claim 13, wherein the dose of the heparinoid derivative for the systemic administration is from about 10 mg to 80 mg and for local administration is from 1 µg to 10 mg:

15. The use of the heparinoid derivatives as claimed in one or more of claims 1 to 4 for the monitoring and diagnosis of the progress of disorders whose course involves an increased activity of metalloproteinases.

16. The use of the heparinoid derivatives as claimed in one or more of claims 1 to 4 for producing a diagnostic test system.

17. The use of a diagnostic test system as set forth in claim 16 for monitoring the result of treatment and functional characterization of disorders.

## Revendications

1. Dérivés d'un héparinoïde,
qui contiennent un héparinoïde choisi dans le groupe constitué par l'énoxaparine, la nadroparine, la fraxiparine, la daltéparine, la fragmine, la bémiparine, la tinzaparine et l'ardéparine,
un agent chélateur qui est lié par covalence à cet héparinoïde, choisi dans le groupe constitué par le dianhydride diéthylènetriamine-N,N,N',N",N"-pentaacétique, l'acide 1,2-bis(2-aminoéthoxyéthane)-N,N,N',N'-tétraacétique, l'acide éthylènediamine-N,N,N',N'-tétraacétique, l'acide 1,4,7,10-tétraazacyclododécane-1,4,7,10-tétraacétique,
l'acide 1,4,7,10-tétraazacyclododécane-1,4,7-triacétique, l'acide nitrilotriacétique, l'acide triéthylènetétraminehexaacétique, l'acide 4-carboxy-5,8,11-tris(carboxyméthyl)-1-phényl-2-oxa-5,8,11-triazatridécan-13-oïque et l'acide N,N'-bis(pyridoxal-5-phosphate)-éthylènediamine-N,N'-diacétique et
un cation métallique paramagnétique choisi dans le groupe des métaux de transition Sc, Ti, V, Cr, Mn, Fe, Co, Ni, Cu, Mo, Ru ou des lanthanides La, Ce, Pr, Nd, Pm, Sm, Eu, Gd, Tb, Dy, Ho, Er, Tm, Yb.

2. Dérivé d'héparinoïde selon la revendication 1, **caractérisé en ce qu'**on utilise comme héparinoïde l'énoxaparine.

3. Dérivé d'un héparinoïde selon la revendication 1 ou 2, **caractérisé en ce que** le cation métallique paramagnétique est Gd³⁺.

4. Dérivé d'héparinoïde selon une ou plusieurs des revendications 1 à 3, **caractérisé en ce que** la teneur en élément de transition ou en lanthanide va de 1 mole par mole à la fonctionnalisation maximale possible du dérivé d'héparinoïde utilisé, de préférence de 1 mole à 20 moles par mole d'énoxaparine.

5. Procédé pour la préparation des dérivés d'héparinoïdes selon une ou plusieurs des revendications 1 à 4, **caractérisé en ce qu'**on fait réagir l'héparinoïde avec un agent chélateur activé, pour aboutir à un chélate d'héparinoïde et ensuite on ajoute l'élément de transition ou le lanthanide.

6. Procédé selon la revendication 5, **caractérisé en ce que** la forme activée de l'agent chélateur est utilisée en un excès de 1:1 à 50:1, de préférence de 1,5:1 à 15:1, par rapport à l'héparinoïde disposé au préalable, sur la base de la masse moléculaire.

7. Procédé selon la revendication 5 ou 6, **caractérisé en ce que** la forme activé de l'agent chélateur est un anhydride tel que le dianhydride diéthylène-triamine-N,N,N',N",N"-pentaacétique.

8. Procédé selon une ou plusieurs des revendications 5 à 7, **caractérisé en ce que** Gd³⁺ est utilisé sous forme de ses sels chlorure de gadolinium(III) hexahydraté ou acétate de gadolinium(III) hydraté.

9. Procédé selon une ou plusieurs des revendications 5 à 8, **caractérisé en ce que** le rapport du chélate d'héparinoïde à l'élément de transition ou au lanthanide dans l'addition est choisi de 1:1 à 1:50, de préférence de 1:1,5 à 1:15, sur la base de la masse moléculaire.

10. Médicament **caractérisé par** une teneur efficace en au moins un dérivé d'héparinoïde selon une ou plusieurs des revendications 1 à 4, conjointement avec un véhicule, additif et/ou autres principes actifs et adjuvants, pharmaceutiquement appropriés et physiologiquement acceptables.

11. Utilisation des dérivés d'héparinoïdes selon une ou plusieurs des revendications 1 à 4, pour la fabrication d'un médicament destiné à la prophylaxie et la thérapie de maladies qui sont **caractérisées par** une activité catabolique accentuée de protéinases, telles que des arthropathies dégénératives, des ostéo-arthroses, des spondyloses, la chondroporose après traumatisme articulaire ou relativement longue immobilisation d'articulations après blessures du ménisque ou de la rotule ou déchirures des ligaments, des maladies du tissu conjonctif telles que les collagénoses ou les maladies périodontaires, les troubles de la cicatrisation ou des maladies chroniques de l'appareil moteur, telles que les arthrites, arthropathies, myalgies, aiguës et chroniques d'origine inflammatoire, immunologique ou métabolique, ou des troubles du métabolisme du tissu osseux.

12. Utilisation des dérivés d'héparinoïdes selon une ou plusieurs des revendications 1 à 4, pour la fabrication d'un médicament destiné à la thérapie et la prophylaxie antithrombotique, comme la prévention de thromboses veineuses, pour empêcher l'apparition de thromboses veineuses chez des patients en chirurgie au stade post-opératoire, pour la prévention d'accidents thrombotiques artériels, notamment dans le cas d'un infarctus du myocarde, dans l'angor instable ou l'angor récidivant, dans l'utilisation après l'angiographie et dans la thérapie de la sténose et de la resténose, dans la thérapie tumorale et de métastases, dans la thérapie de maladies inflammatoires, pour le traitement d'ischémies dans des infarctus cérébraux ou du myocarde, dans la thérapie de maladies du système nerveux central, dans la thérapie lors de transplantations, dans la thérapie de l'asthme ou dans la thérapie de l'angiogenèse.

13. Utilisation selon les revendications 11 et 12, **caractérisée en ce que** l'administration des dérivés d'héparinoïdes s'effectue par voie parentérale comme par injection sous-cutanée, intra-articulaire, intrapéritonéale ou intraveineuse.

14. Utilisation selon la revendication 13, **caractérisée en ce que** la dose du dérivé d'héparinoïde pour l'administration systémique va d'environ 10 mg à 80 mg ou, pour l'application locale, de 1 µg à 10 mg.

15. Utilisation des dérivés d'héparinoïdes selon une ou plusieurs des revendications 1 à 4, pour la surveillance et le diagnostic, de l'évolution de maladies, dans des maladies à l'évolution desquelles participe une activité accrue de métalloprotéinases.

16. Utilisation des dérivés d'héparinoïdes selon une ou plusieurs des revendications 1 à 4, pour la production d'un système de test de diagnostic.

17. Utilisation d'un système de test de diagnostic selon la revendication 16, pour la surveillance du succès d'un traitement et la caractérisation fonctionnelle dans des maladies.
